**Europäisches Patentamt**

(19) **European Patent Office**

**Office européen des brevets**

(11) Veröffentlichungsnummer: **0 080 673**
**B1**

(12) **EUROPÄISCHE PATENTSCHRIFT**

(45) Veröffentlichungstag der Patentschrift:
**29.05.85**

(51) Int. Cl.⁴: **A 61 K 9/20,** A 61 K 31/715

(21) Anmeldenummer: **82110727.3**

(22) Anmeldetag: **20.11.82**

(54) **Oral applizierte Zubereitungsformen von Guar-Mehl.**

(30) Priorität: **28.11.81 DE 3147268**
**11.03.82 DE 3208768**

(43) Veröffentlichungstag der Anmeldung:
**08.06.83 Patentblatt 83/23**

(45) Bekanntmachung des Hinweises auf die Patenterteilung:
**29.05.85 Patentblatt 85/22**

(84) Benannte Vertragsstaaten:
**AT BE CH DE FR GB IT LI LU NL SE**

(56) Entgegenhaltungen:
**GB - A - 1 201 014**
**GB - A - 2 030 583**
**US - A - 3 946 110**

(73) Patentinhaber: **Boehringer Mannheim GmbH,**
**Sandhoferstrasse 116, D-6800 Mannheim 31 (DE)**

(72) Erfinder: **Stemmle, Berthold, Dr. rer. nat.,**
**Beethovenstrasse 16, D-6832 Hockenheim (DE)**
Erfinder: **Wirl, Alexander, Ing. grad., Kurpfalzstrasse 14,**
**D-6711 Heuchelheim (DE)**
Erfinder: **Demmer, Fritz, Dr. rer. nat., Kastanienweg 21,**
**D-6945 Hirschberg-Leutershausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft orale Zubereitungsformen von Guar-Mehl in Form von Tabletten, die dadurch gekennzeichnet sind, daß diese durch trockene Verpressung aus Guar-Mehl einer Korngröße von 60—500 μm unter Zusatz von 5 bis 30% hochdispersem Kieselgel hergestellt sind, und Verfahren zu ihrer Herstellung.

Guar-Mehl (Guar-Gum) ist ein natürliches Hydrokolloid, welches im Endosperm des Samens der Guar-Pflanze (Cyamopsis tetragonoloba) vorkommt und ein Polysaccharid aus Galactose- und Mannosebausteinen darstellt. Qualität und Feinheit des durch Mahlung des von Schale und Keimblättern getrennten Endosperms gewonnenen Guar-Mehls bestimmt sich weiterhin durch die physikalische und mechanische Vor- und Nachbehandlung. Guar-Mehl wird üblicherweise als Verdickungsmittel für Appreturen, als Emulgator, zur Papierverleimung, als Zusatz zu Flotationsmitteln und in gewissem Umfang auch als Dickungs- und Geliermittel beispielsweise bei der Herstellung von Eiscreme verwendet. (Vergleiche Römpps Chemie-Lexikon, 7. Auflage, Seite 1354.)

Es ist beispielsweise aus Jenkins et al. (The Lancet, 1975, 1116, und 1977, 779) bekannt, daß Guar-Mehl, wenn es Patienten in größeren Mengen appliziert wird, den Blutcholesterin- und Glukosespiegel signifikant senkt. Guar-Mehl kann deshalb bei der Behandlung von Hyperglykämien und Hyperlipoproteinämien eingesetzt werden. Die Verabreichung der erforderlichen größeren Mengen (10 bis 30 g pro Tag in 3 bis 5 Dosierungen) stellt jedoch ein erhebliches Problem dar, da Guar-Mehl sehr hydrophil ist und in Mischung mit Wasser rasch Gele bildet. Bei direkter Applikation als trockenes Pulver würde das Guar-Mehl deshalb Mund- und Rachenraum in unangenehmer Weise verkleben und kann sogar durch Nachquellen im Schlund zu Verstopfungen und schweren gesundheitlichen Störungen führen. Die Mischung mit kleineren Mengen Wasser führt zur Bildung klebriger Klumpen, die sich aufgrund der hohen Viskosität der Oberflächenschicht nur noch sehr langsam lösen. Nur wenn das Guar-Mehl unter heftigem Rühren in die mindestens hundertfache Wassermenge eingetragen wird, erhält man eine homogene Mischung. Aufgrund der hohen Viskosität ist dieses Gel schlecht zu schlucken und wird von vielen Patienten als unangenehm empfunden.

In den britischen Patentanmeldungen 2 021 948 und 2 030 583 werden Guar-Granulate beschrieben, bei denen durch Überzug der Guar-Partikel mit Stärke oder Proteinen oder durch spezielle Auswahl des verwendeten Guar-Mehls (Partikelgröße 100 bis 1000 μm) und Granulierung dieser Substanzen mit einer unzureichenden Menge von Wasser ein Granulat erzeugt wird, welches sich leicht in einer größeren Menge Wasser suspendieren läßt, aber erst nach einigen Minuten zu einem hochviskosen Gel durchquillt, so daß diese Mischung getrunken werden kann. Ein ähnliches Präparat ist aus der EP-A-007 619 bekannt, wo die Guarteilchen mit einer Gelatineschicht umhüllt und gegebenenfalls zusätzlich mit einem alkalischen Puffer getränkt werden sollen. Das Präparat ist ebenfalls in Wasser suspendierbar und kann so appliziert werden, da die retardierten Teilchen erst nach einigen Minuten bzw. im sauren Milieu des Magens zu einem viskosen Gel aufquellen. Produkte dieser Art sind jedoch für eine trockene Applikation bzw. eine Applikation mit wenig Flüssigkeit nicht geeignet, da die Aufquellung solcher Granulate trotz der Retardierung bereits teilweise im Mund stattfindet.

Aus der EP-A-005 977 ist weiter bekannt, Guar-Mehl mit entsprechenden Zusätzen zu Brot oder Keksen zu verbacken. Abgesehen davon, daß der Backprozeß das Guar-Mehl schädigt und teilweise unwirksam macht, enthält solches »Brot« üblicherweise nur 5—20% Guar, so daß aus diätischen Gründen unerwünscht große Mengen der Mischung (ca. 200 g) täglich appliziert werden müssen.

Für die Verwendung als Arzneimittel stellte sich deshalb die Aufgabe, Guar-Mehl in eine oral applizierbare Form zu bringen, die auch in größeren Mengen ohne Schwierigkeiten vom Patienten eingenommen werden kann. Aufgrund der Viskosität von Lösungen kam dazu nur eine trockene Form in Frage, die einerseits nicht zu Verklebungen im Mund- und Rachenraum führt, andererseits jedoch im Magen rasch gelöst bzw. durchgequollen wird, so daß das Mittel voll wirksam wird, ohne daß sich von einer viskosen Schleimhülle umgebene trockene Klumpen bilden. Wünschenswert war darüber hinaus noch ein möglichst geringer Anteil an Hilfsstoffen, um die ohnehin großen Substanzmengen nicht unnötig zu vergrößern. Die Auswahl der Hilfsstoffe ist weiterhin dadurch eingeschränkt, daß das Produkt über längere Zeiträume häufig appliziert werden muß und deshalb einen neutralen oder angenehmen Geschmack besitzen sollte. Das Produkt sollte darüber hinaus trocken bzw. mit wenig Flüssigkeit applizierbar sein und keinen oder nur einen sehr geringen pulvrigen Anteil enthalten, der im Mund zum Verkleben neigen würde.

Es hat sich herausgestellt, daß eine der Tablettierung vorhergehende feuchte Granulierung, wie sie in der obenerwähnten Literatur für notwendig gehalten wird, zu einer so starken Verminderung des Quellvermögens führt, daß entsprechende Tabletten nur noch in größere Granulatpartikel zerfallen, die sich mit einer viskosen Schleimschicht aus Guar umhüllen und sich im Laufe der Magen-Darmpassage praktisch nicht mehr lösen. Auch eine Verarbeitung mit zusätzlichen Hilfsstoffen, die als Tablettensprengmittel bzw. zur Erhöhung der Quellfähigkeit von Tabletten bekannt sind, wie Polyvinylpyrrolidon (quervernetzt), Natriumcarboxymethylstärke, Maisstärke, mikrokristalliner Cellulose etc., führt weder nach vorhergehender Granulation noch bei direkter Verpressung zu Tabletten zu befriedigenden Produkten, da entweder bei höherem Preßdruck harte, nicht genügend quellfähige Tabletten entstehen oder bei geringem Preßdruck die Tabletten zwar ein genügendes Quellvermögen aufweisen, jedoch eine so geringe Härte haben, daß die Tabletten bei der weiteren Handhabung zerbrechen bzw. einen zu großen Pulverabrieb aufweisen. Unter Zusatz von Stärke in einer Menge bis zu 20% lassen sich überhaupt keine Tabletten herstellen, da bis zu einer Preßkraft von 7000 N die Formlinge wieder in Pulver zerfallen.

Überraschenderweise lassen sich ausreichend harte, nicht zerbrechende Guar-Tabletten jedoch herstellen, wenn das Guar-Mehl trocken mit 5—30 Gew.-%, vorzugsweise 10—15% hochdispersem Kieselgel vermischt und trocken zu Tabletten verpreßt wird.

Überraschenderweise hat sich weiterhin herausgestellt, daß sehr feines Guar-Mehl, welches sich bekanntermaßen besonders gut und rasch in Wasser löst, für den erfindungsgemäßen Zweck weniger geeignet ist als ein gröberes Material, welches sich wesentlich langsamer löst, da feine Partikel (kleiner 60 µm) offensichtlich bei höheren Preßdrücken zusammenfließen und die Partikel des zugesetzten Kieselgels mehr oder weniger umhüllen, wodurch der Zerfall der Tabletten in Einzelpartikel und damit die Quellfähigkeit bei hohen Preßdrücken rasch abnimmt. Andererseits scheinen Partikel mit einer Größe von über 60 µm, vorzugsweise einer Größe von etwa 60 bis 500 µm auch bei höheren Preßdrücken nicht ineinander zu fließen, so daß auf diese Art und Weise hergestellte Tabletten ein Quellvermögen haben, das relativ unabhängig von dem verwendeten Preßdruck ist. Es lassen sich aus solchem Guar-Mehl deshalb für die orale Applikation besonders geeignete, harte und abriebfeste Tabletten herstellen, die trotzdem noch ein genügend hohes Quellvermögen besitzen.

Erwartungsgemäß ist die Quellfähigkeit der hergestellten Tabletten um so höher, je höher der Anteil an zugesetztem Kieselgel ist und je geringer der verwendete Preßdruck ist, d. h., je geringer die Härte der erzeugten Tabletten ausfällt. Andererseits wächst natürlich mit abnehmender Härte auch der Pulverabrieb bei der Handhabung solcher Tabletten.

Gegebenenfalls können der Rezeptur zum besseren Tablettieren noch 1—5% eines üblichen Bindemittels wie Polyethylenglykol, Stärke oder Polyvinylpyrrolidon zugesetzt werden.

Da die erfindungsgemäßen Zubereitungsformen trocken bzw. mit einer geringen Menge Flüssigkeit zum Nachspülen geschluckt werden sollen, hat es sich als vorteilhaft erwiesen, statt einer großen Tablette eine Mehrzahl von kleineren Tabletten zu applizieren. Aus herstellungstechnischen Gründen werden nun die Tabletten mit einem Durchmesser von 3—5 mm und einer Höhe von 1—3 mm bevorzugt. Wegen des geringen Hilfsstoffanteils dieser Präparate genügen dann 100—200 solcher Tabletten für jede Dosierung. Durch eine dünne Drageedecke aus Zucker oder einem filmbildenden wasserlöslichen Polymeren kann die Applikation weiter gefördert werden.

Bei Versuchen, die Akzeptanz solcher Tabletten noch zu verbessern, indem diesen Tabletten Aromastoffe, Säuren und Süßstoffe zugesetzt wurden, um nicht nur einen neutralen, sondern auch einen angenehmen Geschmack zu erzeugen, soweit die Einnehmenden die Tabletten zerkauen, traten jedoch unerwarteterweise Schwierigkeiten auf. Es zeigt sich, daß bei der Lagerung solcher Produkte bereits nach 6 Wochen bei 35°C ein Strukturabbau des Guar-Mehls eintritt, der zu einer Verringerung des Quellvermögens und der Quellungsgeschwindigkeit führt. Ob gleichzeitig ein geschmacklicher Abbau eintritt, kann neben der Aromatisierung nicht festgestellt werden.

Da Quellvermögen und Quellungsgeschwindigkeit für die pharmakologische Wirksamkeit wichtige Qualitätskriterien sind, war es notwendig, eine Aromatisierung zu finden, die diese Eigenschaften des Guar-Rohstoffes nicht verändern. Weder eine Veränderung der zugesetzten Säuren, versucht wurden alle üblichen Genußsäuren wie Zitronensäure, Äpfelsäure, Weinsäure, Ascorbinsäure, Glukonsäure, Fumarsäure und Bernsteinsäure, noch eine Veränderung der Aromastoffe, wie Zitrusaroma, Himbeer-, Erdbeer-, Heidelbeer-, Aprikosen- oder Kirscharoma, wobei sowohl künstliche als auch natürliche Aromastoffe angewendet werden können, ändert etwas an dem unwillkommenen Abbau des Guar-Mehls.

Es stellte sich deshalb die Aufgabe, andere Zusatzstoffe zu finden, die einerseits den guten Geschmack so aromatisierter Tabletten nicht verändern und andererseits den Abbau des Guar-Mehls verhindern bzw. so verlangsamen, daß er nicht mehr störend wirkt. Überraschenderweise gelingt diese Stabilisierung bereits dadurch, daß man die den frischen Geschmack solcher aromatisierten Rezepturen hervorrufende Säure, die in einer Menge von 1—10% enthalten ist, vor der Zumischung ihrerseits mit einer Menge von 1—20%, vorzugsweise etwa 2—10%, bezogen auf die Säure eines Hydrophobierungsmittels, überzieht. Bezogen auf die Gesamtmischung macht das Überzugsmittel nur eine Menge von 0,01 bis 2% aus. Als Hydrophobierungsmittel werden gesättigte oder ungesättigte feste Fettsäuren, Mono-, Di- oder Triglyceride derselben, natürliche oder synthetische Wachse, Wachsalkohole oder Gemische derselben bevorzugt, jedoch können auch andere hydrophobe Hilfsstoffe, wie Talkum, Magnesiumstearat, Polymere, wie Methacrylsäure oder Methacrylsäureester allein oder in Mischung verwendet werden.

Als Säuren können alle genußfähigen Säuren verwendet werden, wobei die oben bereits aufgezählten wegen ihrer weiten Verbreitung bevorzugt sind.

Die oben aufgeführten Aromastoffe können in einer Menge von 0,5 bis 3%, vorzugsweise etwa 0,8 bis 2% enthalten sein.

Es muß als überraschend bezeichnet werden, daß einerseits der Zusatz von relativ geringen Mengen einer festen Säure in Pulverform in der festen Tablettenrezeptur überhaupt eine Veränderung des Guar-Mehls hervorruft und andererseits, daß durch Zusatz einer noch wesentlich geringeren Menge

3

eines Hydrophobierungsmittels dieser negative Einfluß wieder aufgehoben werden kann.

Da Guar-Mehl in sehr hohen Dosierungen (10—30 g pro Tag) appliziert werden muß, um einen pharmakologischen Effekt auszulösen, müssen Guar-Rezepturen auf der anderen Seite einen ungewöhnlich hohen Anteil an Guar und einen möglichst geringen Anteil an Hilfsstoffen enthalten. Es ist unter diesen Umständen außerordentlich vorteilhaft, daß es auf die oben beschriebene, in den Ansprüchen näher gekennzeichnete Weise gelingt, mit nur geringen zusätzlichen Hilfsstoff-Anteilen stabile Rezepturen zu erzeugen, in denen der Guar-Anteil an der Gesamtrezeptur 50 bis zu über 90% beträgt.

In der folgenden Tabelle sind die relativen dynamischen Viskositäten verschiedener erfindungsgemäß und ohne den erfindungsgemäßen Zusatz hergestellter Tablettenchargen sonst identischer Zusammensetzung und Verarbeitung miteinander verglichen.

Die Bestimmung der relativen dynamischen Viskosität in Abhängigkeit von der Zeit wird mit einem Rotationsviskosimeter der Fa. Contraves AG, Zürich, Type Epprecht Rheomat 15 durchgeführt. Die Quellung der Mini-Tabletten (entspricht 10 g Guar-Gum) erfolgt in einem Rundkolben mit 800 ml Wasser bei 37°C unter Rühren mit einem Paddle-Rührer (90 UpM). In bestimmten Zeitabständen werden Proben entnommen, in den Meßbecher des obengenannten Rotationsviskosimeters übergeführt und die Viskosität bei einer Spindelgeschwindigkeitseinstellung von 10 Skalenteilen bestimmt. Das Meßsystem des Rotationsviskosimeters ist ebenfalls auf 37°C thermostatisiert. Verwendet wird das Meßsystem C.

Die folgenden Beispiele zeigen den Einfluß von Preßdruck, Hilfsstoffzusammensetzung und Guar-Korngröße auf Tablettenhärte und Quellvermögen der entsprechenden Tabletten

Sämtliche in der Beschreibung angegebenen Prozentangaben sind als »Prozentgewicht« zu verstehen.

| Zusammensetzung | (%) | Charge | Relative dynamische Viskosität bei thermischer Belastung 6 Wochen | | | nach 2 h (PA · s) 6 Monate | |
|---|---|---|---|---|---|---|---|
| | | | KS[2]) | 35°C | 45°C | KS[2]) | 35°C |
| I | | | | | | | |
| Guar-Gum | 81,5 | 1 | 1,3 | 0,6 | 0,5 | | |
| Freie Zitronensäure | 3,7 | 2 | 1,2 | 1,1 | 0,8 | 0,8 | 0,3 |
| Indifferente Hilfsstoffe | 14,8 | | | | | | |
| II | | | | | | | |
| Guar-Gum | 81,5 | 3 | 1,4 | 1,2 | 1,2 | | |
| Hydrophobierte Zitronensäure | 3,7 | 4 | 1,3 | 1,2 | 1,3 | | |
| Indifferente Hilfsstoffe | 14,8 | 5 | 1,4 | 1,3 | 1,2 | 1,3 | 1,4 |

Beispiel 1

Quellvermögen und physikalische Parameter der erfindungsgemäßen Rezeptur mit 10% hochdispersem Kieselgel und 2% Bindemittel (Rezeptur I) im Vergleich zu analogen Rezepturen mit mikrokristalliner Cellulose (Rez. II), Polyvinylpyrrolidon (quervernetzt) (Rez. III), Natriumcarboxymethylstärke (Rez. IV) und Maisstärke (Rez. V).

| Rezeptur | Preßkraft (N) | Härte[1]) (N) | Abrieb[2]) (%) | Viskosität $\eta_{rel}$ 0,5 h | in Pa·s nach[3]) 1,0 h |
|---|---|---|---|---|---|
| I | 2000 | 44,1 | 3,4 | 1,046 | 1,199 |
| II | 1450 | 33,8 | 11,93 | 0,270 | 0,270 |

Fortsetzung

| Rezeptur | Preßkraft (N) | Härte[1] (N) | Abrieb[2] (%) | Viskosität $\eta_{rel}$ 0,5 h | in Pa·s nach[3] 1,0 h |
|----------|---------------|--------------|---------------|---------------------------------|-------------------------|
| III | 3000 | 10 | 17,2 | 1,055 | 1,226 |
| IV | 7000 | — | — | — | — |
| V | 7000 | — | — | — | — |

Zusammensetzung der Rezepturen I—V und Herstellungsverfahren

| | Zusammensetzung (%) Rezeptur | | | | |
|--|------|------|------|------|------|
| | I | II | III | IV | V |
| Guar-Gum | 88,24 | 88,24 | 88,24 | 88,24 | 88,24 |
| Macrogol 6000 | 1,96 | 1,96 | 1,96 | 1,96 | 1,96 |
| Hochdisperses Kieselgel | 9,80 | — | — | — | — |
| Mikrokrist. Cellulose | — | 9,80 | — | — | — |
| Poly(vinylpyrrolidon), quervernetzt | — | — | 9,80 | — | — |
| Natriumcarboxymethylstärke | — | — | — | 9,80 | — |
| Maisstärke | — | — | — | — | 9,80 |

Alle Bestandteile werden gemischt und zu »Mini-Tabletten« verpreßt (Durchmesser 5 mm, Höhe 2 mm).

Beispiel 2

Quellvermögen von Guar-Minitabletten der erfindungsgemäßen Rezeptur I mit »feinem« und »grobem« Guar-Gum sowie physikalische Parameter.

| | Härte (N)[1] | Abrieb (%)[2] | Preßkraft (N) | Viskosität $\eta_{rel}$ 0,5 h | in Pa·s nach 1,0 h[3] |
|--|--------------|---------------|---------------|---------------------------------|-------------------------|
| Rezeptur mit »feinem« Guar-Gum kleiner 60 µm | 16 76 | 23 1,2 | 900 2400 | 1,307 — | 1,496 0,270 |
| Rezeptur mit »grobem« Guar-Gum größer 60 µm | 16 64 | 65 2,2 | 1200 2600 | 0,883 0,847 | 1,118 1,118 |

[1] Schleuninger-Tester, Auflage: 5 Minitabletten
[2] Roche Friabilator
[3] Die Bestimmung der relativen dynamischen Viskosität in Abhängigkeit von der Zeit wird mit einem Rotationsviskosimeter der Fa. Contraves AG, Zürich, Type Epprecht Rheomat 15, durchgeführt. Die Quellung der Minitabletten (entsprechend 10 g Guar-Gum) erfolgt in einem Rundkolben mit 800 ml Wasser bei 37°C unter Rühren mit einem Paddle-Rührer (90 UpM). In bestimmten Zeitabständen werden Proben entnommen, in den Meßbecher des o. g. Rotationsviskometers übergeführt und die Viskosität bei einer Spindelgeschwindigkeitseinstellung von 10 Skalenteilen bestimmt. Das Meßsystem des Rotationsviskosimeters ist ebenfalls auf 37°C thermostatisiert. Verwendet wird das Meßsystem C.

**Patentansprüche**

1. Orale Zubereitungsform von Guar-Mehl in Form von Tabletten, dadurch gekennzeichnet, daß diese durch trockene Verpessung aus Guar-Mehl einer Korngröße von 60—500 μm unter Zusatz von 5 bis 30% hochdispersem Kieselgel hergestellt sind.

2. Zubereitungsform gemäß Anspruch 1, dadurch gekennzeichnet, daß die Tablette zusätzlich bis zu 5% eines üblichen Bindemittels enthält.

3. Zubereitungsform gemäß einem der Ansprüche 1 bis 2, dadurch gekennzeichnet, daß die Tabletten einen Durchmesser von 2 bis 5 mm und eine Dicke von 1 bis 4 mm haben und eine Härte von über 30 N aufweisen.

4. Zubereitung gemäß Anspruch 3, dadurch gekennzeichnet, daß mehrere solcher Tabletten in einer Kapsel zu einer Dosiseinheit zusammengefaßt sind.

5. Zubereitungsform nach Ansprüchen 1 bis 4, dadurch gekennzeichnet, daß diese 0,5 bis 3% Aromastoffe und 1 bis 10% einer zum menschlichen Genuß geeigneten Carbonsäure enthält, wobei die Säure mit 1 bis 20% ihres Gewichtes eines Hydrophobierungsmittels überzogen ist.

6. Tablette nach Anspruch 5, dadurch gekennzeichnet, daß als Hydrophobierungsmittel gesättigte oder ungesättigte feste Fettsäuren, Mono-, Di- oder Triglyceride derselben, natürliche oder synthetische Wachse, Wachsalkohole, Talkum, Magnesiumstearat, Polymere, wie Methacrylsäure oder Methacrylsäureester, allein oder in Mischung verwendet werden.

7. Tablette gemäß Anspruch 5 oder 6, dadurch gekennzeichnet, daß als Carbonsäure Zitronensäure, Äpfelsäure, Weinsäure, Ascorbinsäure, Glukonsäure, Fumarsäure und Bernsteinsäure verwendet wird.

8. Verfahren zur Herstellung von Zubereitungsformen gemäß Anspruch 1 bis 7, dadurch gekennzeichnet, daß die Bestandteile trocken vermischt und in einer Tablettenpresse mit einem Preßdruck von 3000 bis 15 000 N/cm$^2$ kompaktiert werden.

**Claims**

1. Oral form of preparation of guar flour in the form of tablets, characterised in that these are prepared by dry pressing from guar flour with a granular size of 60—500 μm, with the addition of 5 to 30% of highly dispersed silica gel.

2. Form of preparation according to claim 1, characterised in that the tablets additionally contain up to 5% of a conventional binding agent.

3. Form of preparation according to one of claims 1 to 2, characterised in that the tablets have a diameter of 2 to 5 mm and a thickness of 1 to 4 mm and a hardness of more than 30 N.

4. Form of preparation according to claim 3, characterised in that several such tablets are combined in a capsule to give a dosage unit.

5. Form of preparation according to claims 1 to 4, characterised in that these contain 0.5 to 3% of aroma materials and 1 to 10% of a carboxylic acid suitable for human consumption, whereby the acid is coated with 1 to 20% of its weight of a hydrophobing agent.

6. Tablet according to claim 5, characterised in that, as hydrophobing agent, there are used saturated or unsaturated solid fatty acids, mono-, di- or triglycerides thereof, natural or synthetic waxes, wax alcohols, talc, magnesium stearate, polymers, such as methacrylic acid or methacrylic acid ester polymers, alone or in mixtures.

7. Tablets according to claim 5 or 6, characterised in that, as carboxylic acid, there is used citric acid, malic acid, tartaric acid, ascorbic acid, gluconic acid, fumaric acid or succinic acid.

8. Process for the preparation of forms of preparation according to claims 1 to 7, characterised in that the components are dry mixed and compacted in a tabletting press with a pressure of 3000 to 15 000 N/cm$^2$.

**Revendications**

1. Forme de préparation de gomme guar pour administration par voie orale en form de comprimés, caractérisée en ce qu'elle est préparée par compression à sec à partir de gomme guar dont les grains ont une taille comprise entre 60 et 500 μm en ajoutant entre 5 et 30% de gel de silice très finement dispersé.

2. Forme de préparation selon la revendication 1, caractérisée en ce que le comprimé renferme en outre jusqu'à 5% d'un liant habituel.

3. Forme de préparation selon la revendication 1 ou 2, caractérisée en ce que les comprimés ont un diamètre compris entre 2 et 5 mm et une épaisseur entre 1 et 4 mm et une dureté supérieure à 30 N.

4. Préparation selon la revendication 3, caractérisée en ce que plusieurs de ces comprimés sont réunis dans une capsule pour former une unité de dosage.

5. Forme de préparation selon les revendications 1 à 4, caractérisée en ce qu'elle contient entre 0,5 et 3% de substances aromatiques et entre 1 et 10% d'un acide carboxylique approprié à la consommation humaine, cet acide étant revêtu par un agent hydrophobe en une quantité comprise entre 1 et 20% du poids de l'acide.

6. Comprimé selon la revendication 5, caractérisé en ce qu'en tant qu'agents hydrophobes sont utilisés: des acides gras saturés ou insaturés solides, leur mono-, di- et triglycérides, des cires naturelles ou synthétiques, des alcools de cires, du talc, du stéarate de magnésium, des polymères comme l'acide méthacrylique ou l'acide méthacrylique estérifié, seuls ou en mélanges.

7. Comprimé selon la revendication 5 ou 6, caractérisé en ce qu'en tant qu'acide carboxylique sont utilisés les acides citrique, malonique, tartrique, ascorbique, gluconique, fumarique et succinique.

8. Procédé pour la fabrication de formes de préparation selon les revendications 1 à 7, caractérisé en ce que les ingrédients sont mélangés à sec et sont compactés dans une presse à faire des comprimés sous une pression comprise entre 3000 et 15 000 N/cm².